# EUROPÄISCHE PATENTSCHRIFT

(11) **EP 3 334 475 B1**
(45) Veröffentlichungstag und Bekanntmachung des Hinweises auf die Patenterteilung: **04.03.2020**
(21) Anmeldenummer: 16754189.5
(22) Anmeldetag: 09.08.2016
(51) Int. Cl.: A61M 1/28, A61M 5/14, F16M 11/08, F16M 11/10, A61M 1/16

(54) **PERITONEALDIALYSEGERÄT**
PERITONEAL DIALYSIS DEVICE
APPAREIL DE DIALYSE PÉRITONÉALE

(30) Priorität: 11.08.2015 DE 102015010431
(43) Veröffentlichungstag der Anmeldung: 20.06.2018
(73) Patentinhaber: Fresenius Medical Care Deutschland GmbH, 61352 Bad Homburg (DE)
(72) Erfinder: WIESKOTTEN, Sebastian, 64560 Erfelden (DE); ZEYHER, Peter, 64289 Darmstadt (DE); GÜNTHER, Florian, 09599 Freiburg (DE); WOLF, Klaus, 97450 Arnstein-Müdesheim (DE)
(74) Vertreter: Herrmann, Uwe
(86) Internationale Anmeldenummer: PCT/EP2016/001364
(87) Internationale Veröffentlichungsnummer: WO 2017/025186

(56) Entgegenhaltungen:
- EP-A1- 2 682 139
- US-A1- 2009 212 178
- US-A1- 2009 299 273
- US-A1- 2014 014 786
- US-A1- 2014 097 303
- US-B2- 7 905 855

## Beschreibung

Die vorliegende Erfindung betrifft ein Peritonealdialysegerät mit wenigstens einer Aufnahme zur Fixierung eines oder mehrerer Abschnitte eines Schlauchsets zur Leitung von Dialyselösung von und/oder zu dem Patienten.

Bei aus dem Stand der Technik bekannten Peritonealdialysegeräten ist es bekannt, an dem Gerät eine Aufnahme z.B. für Teile eines Schlauchsets, zur Aufnahme von Konnektoren oder Verschlusskappen oder von Patientenanschlussleitungen etc. vorzusehen. So muss beispielsweise im Rahmen einer Peritonealdialysetherapie der Patient die Patientenanschlussleitung unter aseptischen Bedingungen an eine Aufnahmevorrichtung des Peritonealdialysegerätes anschließen.

Dazu ist es bekannt, eine Aufnahme, d.h. einen so genannten Organizer, zu verwenden, in dem z.B. der Konnektor einer Patientenanschlussleitung fixiert werden kann, sodass der Patient mit nur einer Hand eine Fluidverbindung herstellen kann.

Aus dem Stand der Technik sind Peritonealdialysegeräte mit Vorrichtungen zur Aufnahme von Schläuchen bekannt. Beispielsweie offenbart die Druckschrift US2014/0097303A1 eine Vorrichtung zur Aufnahme und Ausrichtung medizinischer Fluidleitungen. Zudem ist aus der Druckschrift US2009/0212178A1 eine Standvorrichtung für medizinische Fluidbeutel bekannt. Darüberhinaus offenbart die Druckschrift US2009/0299273A1 ein tragbares, flexibles Peritonealdialysegerät.

Beim Einlegen des Schlauches bzw. des Konnektors in die Aufnahme des Peritonealdialysegerätes treten Kräfte, insbesondere vertikale Kräfte auf, die von dem mit der Aufnahme verbundenen Teil des Gerätes aufgenommen werden müssen.

Insbesondere für den Fall, dass die Aufnahme an einem Hebel angeordnet ist, der vergleichsweise lang ausgeführt ist, entsteht dabei ein großes Moment, das zu einer Beschädigung des üblicherweise aus Kunststoff bestehenden Gehäuses des Peritonealdialysegerätes führen kann.

Der vorliegenden Erfindung liegt daher die Aufgabe zugrunde, ein Peritonealdialysegerät der eingangs genannten Art dahingehend weiterzubilden, dass auch beim Auftreten vergleichsweise großer Kräfte oder Momente auf die Aufnahme eine Beschädigung des Gerätes und insbesondere des Gerätegehäuses vermieden wird.

Diese Aufgabe wird durch ein Peritonealdialysegerät mit den Merkmalen des Anspruchs 1 gelöst. Danach ist vorgesehen, dass zumindest eine erste Schwenkachse vorgesehen ist, um die die Aufnahme von einer ersten Position in eine zweite Position verschwenkbar ist.

Dadurch lässt sich der Vorteil erreichen, dass bei Überschreiten einer bestimmten Kraft oder eines bestimmten Momentes die Aufnahme von einer ersten in eine zweite Position verschwenkt wird und somit die auf das Gehäuse wirkenden Kräfte bzw. Momente verringert werden. Die erste Schwenkachse erfüllt somit die Aufgabe einer Überlastsicherung, die eine Beschädigung des Gehäuses oder eines sonstigen Elementes des Peritonealdialysegerätes, an dem die Aufnahme angeordnet ist, verhindert.

In einer bevorzugten Ausgestaltung der Erfindung weist das Peritonealdialysegerät wenigstens ein Element auf, bei dem es sich vorzugsweise um das Gerätegehäuse handelt, wobei die Aufnahme unmittelbar an diesem Element angeordnet ist und wobei sich die erste Schwenkachse an dem Element bzw. an dem Gehäuse befindet.

Von der Erfindung ist jedoch auch der Fall umfasst, dass das Peritonealdialysegerät ein Element bzw. ein Gehäuse aufweist, und dass die Aufnahme nicht unmittelbar daran befestigt ist, sondern mittelbar über wenigstens eine Halterung. In diesem Fall kann sich die erste Schwenkachse zwischen der Halterung und der Aufnahme und/oder zwischen der Halterung und dem Element bzw. dem Gerätegehäuse befinden.

In allen Fällen ist sichergestellt, dass die Aufnahme zu großen Kräften "ausweicht", indem sie um die erste Schwenkachse verschwenkt wird, wenn bestimmte Kräfte oder Momente überschritten werden.

Vorzugsweise verläuft die erste Schwenkachse horizontal oder im Wesentlichen horizontal. Auch andere Orientierungen der ersten Schwenkachse sind denkbar und von der Erfindung mit umfasst. Wesentlich ist, dass die erste Schwenkachse ein Ausweichen der Aufnahme bei zu großen Kräften bzw. Momenten ermöglicht und somit eine Beschädigung des Elementes des Gerätes verhindert, mit dem die Aufnahme verbunden ist.

In einer weiteren Ausgestaltung der Erfindung ist die Aufnahme in ihrer ersten, d.h. nicht verschwenkten Position horizontal oder im Wesentlichen horizontal ausgerichtet. Ausgehend von dieser Position kann sie mittels der ersten Schwenkachse z.B. in eine vertikale oder schräg nach unten gerichtete Position verschwenkt werden.

In einer weiteren Ausgestaltung der Erfindung ist vorgesehen, dass das Peritonealdialysegerät wenigstens ein Element vorzugsweise wenigstens ein Gehäuse aufweist, an dem sich zumindest eine zweite Schwenkachse befindet, die vorzugsweise nicht parallel zur ersten Schwenkachse verläuft und um die die Aufnahme oder eine mit der Aufnahme in Verbindung stehende Halterung verschwenkbar ist. So ist es möglich, die Aufnahme um diese zweite Schwenkachse z.B. von einem Bereich neben dem Gehäuse vor das Gehäuse zu drehen.

Diese zweite Schwenkachse kann vertikal oder im Wesentlichen vertikal verlaufen. Auch andere Orientierungen der zweiten Schwenkachse sind denkbar und von der Erfindung mit umfasst.

Vorzugsweise ist die zweite Schwenkachse unterhalb des Gerätegehäuses angeordnet.

Um sicherzustellen, dass die Aufnahme erst bei Überschreiten einer bestimmten auf diese wirkenden Kraft oder Moment von der ersten in die zweite Position verschwenkt wird, können Halterungsmittel vorhanden sein, die so ausgebildet sind, das sie ein Verschwenken in die zweite Position erst dann ermöglichen, wenn die auf die Aufnahme wirkende Kraft bzw. das auf diese wirkende Moment einen Grenzwert übersteigt. Auf diese Weise wird vermieden, dass in der nicht verschwenkten Position eine unzulässige Kraft z.B. von oben auf die Aufnahme bzw. auf die Halterung wirkt, die auf das Gerätegehäuse übertragen wird.

Bei den genannten Halterungsmitteln kann es sich um wenigstens zwei Teile handeln, die durch eine kraft- oder formschlüssige Verbindung miteinander in Verbindung stehen oder auch um einen oder mehrere Magneten.

Beispielsweise kann ein Klappmechanismus in Form von Scharnieren, Magneten oder Rastvorrichtungen vorgesehen sein, sodass der Organizer bzw. die Aufnahme nach unten abklappen kann.

An dieser Stelle wird darauf hingewiesen, dass der Begriff "Schwenkachse" jeden beliebigen Mechanismus, ein- oder mehrteilig, mit einer oder mehreren Schwenkachsen umfasst, um die die Aufnahme verschwenkt bzw. abgeklappt werden kann. Beispiele sind die Verwendung eines oder mehrerer Bolzen, die in Lagern aufgenommen sind, ein Hebelmechanismus, Vorsprünge, die in Ausnehmungen aufgenommen sind etc. Auch ein Filmscharnier ist denkbar und von der Erfindung mitumfasst.

Weitere Einzelheiten und Vorteile der Erfindung werden anhand eines in der Zeichnung dargestellten Ausführungsbeispiels näher erläutert.

Es zeigen:
- Figur 1:: eine perspektivische Ansicht eines Peritonealdialysegerätes gemäß der Erfindung,
- Figur 2:: eine Detailansicht des Gehäuses mit Halterung und Aufnahme,
- Figur 3:: eine weitere Detailansicht des Gehäuses mit Halterung und Aufnahme,
- Figur 4:: eine schematische Ansicht der erfindungsgemäßen Anordnung in der ersten Position und in der zweiten Position der Aufnahme.

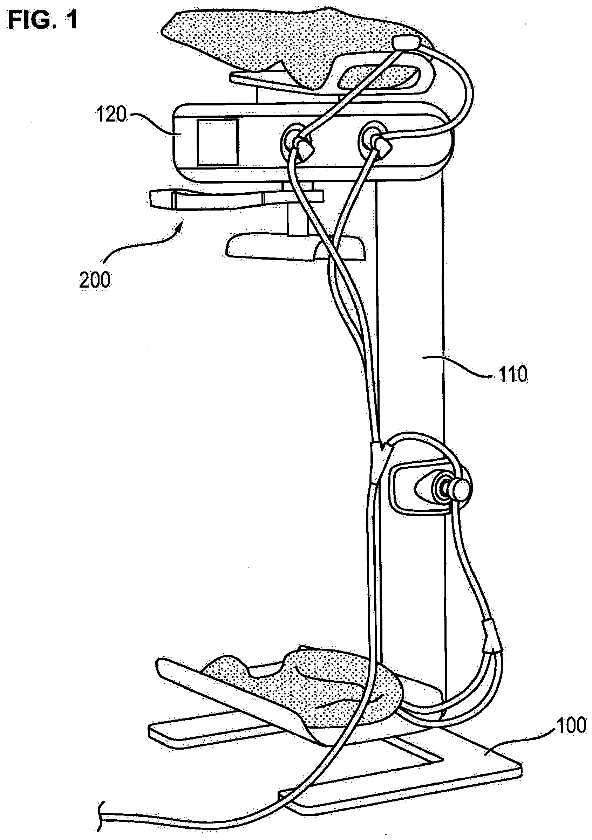

Figur 1 zeigt ein Peritonealdialysegerät gemäß der Erfindung in einer perspektivischen Ansicht.

Das Gerät weist einen Gerätefuß 100 auf, sowie einen sich von diesen nach oben erstreckenden Gehäuseträger 110. Das Bezugszeichen 120 kennzeichnet das Gerätegehäuse, das an dem Gehäuseträger 110 angeordnet ist. In dem Gerätegehäuse 120 befindet sich die für den Betrieb des Gehäuses notwendige Steuerung und ggf. Anzeige und/oder Bedienelemente.

Oberhalb des Gerätegehäuses 120 befindet sich eine Wägeschale, in die Lösungsbeutel mit zu dem verabreichendem Dialysat eingelegt werden können.

Unterhalb des Gerätegehäuses 120 und unmittelbar oberhalb des Fußes 100 befindet sich die Aufnahmeschale, in der Beutel angeordnet sind, die mit verbrauchtem Dialysat befüllt werden.

Das Bezugszeichen 200 kennzeichnet eine Anordnung, die die erfindungsgemäße Aufnahme umfasst, die im Folgenden näher beschrieben wird.

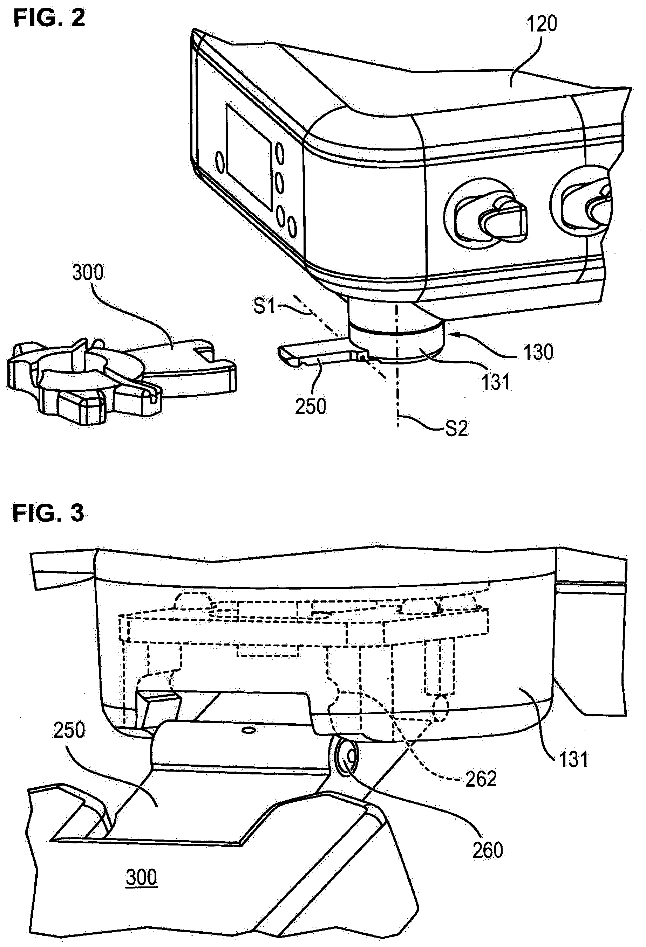

Figur 2 zeigt einen Teilbereich des Gerätegehäuses 120.

Wie dies aus Figur 2 ersichtlich ist, befindet sich in einem Eckbereich auf der Unterseite des Gerätegehäuses ein Vorsprung 130, dessen unterer Teil 131 relativ zu dem Gehäuse 120 um die vertikale Schwenkachse S2 verdrehbar ist.

An diesem Element 131 befindet sich die Halterung 250. Die Halterung 250 erstreckt sich in der in Figur 2 dargestellten Position in horizontaler Richtung von dem Element 131 weg.

Sie ist relativ zu dem Element 131 um eine horizontal verlaufende erste Schwenkachse S1 verschwenkbar.

Mit dieser Halterung 250 ist beispielsweise durch Kraft- oder Formschluss die Aufnahme 300 verbindbar, die im Folgenden auch als Organizer bezeichnet wird. Dieser Organizer dient zur Aufnahme von einem oder mehreren Bestandteilen eines Schlauchsets eines Peritonealdialysegerätes. Dazu gehören beispielsweise die Schläuche selbst, sowie auch Konnektoren zum Verbinden zweier Schlauchabschnitte.

So ist es beispielsweise denkbar, dass diese Aufnahme dazu verwendet wird, den Schlauch, der mit dem Dialysebeutel verbunden ist mit dem Patientenschlauch zu verbinden, der in die Bauchhöhle führt. Dazu können an beiden Enden Konnektoren vorgesehen sein, die vorzugsweise formschlüssig in der Aufnahme 300 angeordnet sind, sodass der Patient nur eine Hand braucht, um eine entsprechende Fluidverbindung herzustellen.

Grundsätzlich ist die Aufnahme nicht genau auf diese Verwendung beschränkt, sondern kann jedes beliebige geeignete Element eines Schlauchsets eines Peritonealdialysegerätes aufnehmen.

An dieser Stelle wird darauf hingewiesen, dass die vorliegende Erfindung das Peritonealdialysegerät als solches, d.h. ohne Schlauchset, sowie auch ein Peritonealdialysegerät unter Schutz stellt, in dem sich ein solches Schlauchset befindet.

Wie dies oben ausgeführt wurde, ist die Halterung 250 unterhalb des Gerätegehäuses 120 schwenkbar angeordnet. So kann sie beispielsweise von einer Position, in der sie in eine Richtung neben das Gehäuse 120 weist, nach vorne verdreht werden. Zum Bestücken mit dem Disposable und auch während der Behandlung kann der Organizer bzw. die Aufnahme 300 z.B. vor das Gerät, d.h. gemäß Figur 2 nach vorne verdreht werden.

Wird nun die Aufnahme 300 mit Elementen des Schlauchsets, wie beispielsweise mit einem Konnektor bestückt, wirken auf die Aufnahme 300 Kräfte, im Allgemeinen von oben.

Diese Kräfte werden dann über die Halterung 250 auf das Element 131 und von diesem auf das Gehäuse 120 übertragen. Dabei kann es zu unzulässigen Krafteinleitungen in das üblicherweise aus Kunststoff bestehende Gehäuse 120 kommen, das dann ggf. beschädigt wird.

Um eine solche Beschädigung zu vermeiden, ist vorgesehen, dass die Aufnahme bzw. die Halterung 250 schwenkbar an dem Gehäuse 120 angeordnet ist. Drückt eine unzulässig große Kraft von oben auf die Aufnahme 300, wird die Halterung 250 mit der Aufnahme 300 nach unten verschwenkt und weicht somit aus. Der Organizer klappt somit nach unten ab.

Statt einer einfachen Schwenkachse kann auch ein beliebiger anderer Klappmechanismus, Hebelmechanismus oder sonstiger Mechanismus verwendet werden, um ein solches Abklappern bzw. Verschwenken der Aufnahme 300 zu ermöglichen.

Figur 3 zeigt eine Detaildarstellung des Elementes 131, das um die zweite Schwenkachse S2 relativ zum Gehäuse verdrehbar ist. An diesem Element 131 befindet sich um die erste Schwenkachse S1 verschwenkbar die Halterung 250, wie dies in Figur 2 ausgeführt wurde.

Um ein ungewolltes Herabschwenken der Halterung 250 und somit auch des Organizers 300 zu verhindern, ist ein Haltemechanismus vorgesehen, der beispielsweise in Form einer Rastvorrichtung ausgeführt sein kann. Das Bezugszeichen 260 in Figur 3 kennzeichnet einen federbelasteten Kolben, Kugel oder sonstigen Vorsprung, der in der ersten Position der Aufnahme in eine entsprechende Aufnahme 262 eingedrückt wird. Übersteigt die auf die Aufnahme wirkenden Kraft einen bestimmten Grenzwert, wird der Kolben 260 etc. entgegen der Federkraft eingedrückt, woraufhin die Halterung 250 nach unten verschwenkt werden kann.

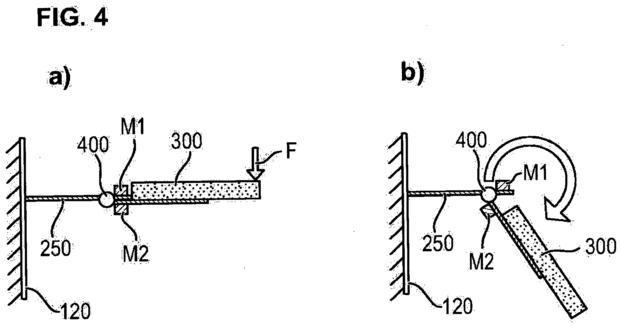

Figur 4 zeigt ein weiteres Ausführungsbeispiel eines solchen Halterungsmechanismus.

In Figur 4 ist mit den Bezugszeichen 120 schematisch das Gehäuse eines Peritonealdialysegerätes gekennzeichnet.

Vor diesem erstreckt sich in horizontaler Richtung die Halterung 250. Am von dem Gehäuse 120 abgewandten Bereich bzw. Endbereich der Halterung 250 befindet sich ein Scharnier 400, um das die Aufnahme 300 von der ersten Position gemäß Figur 4 a) in die zweite Position gemäß Figur 4 b) nach unten verschwenkt werden kann. Dies ist durch den Pfeil gemäß Figur 4 b) angedeutet.

In dem Ausführungsbeispiel gemäß Figur 4 wird die Fixierung der Aufnahme 300 in der ersten Position, d.h. in der horizontalen Position nicht durch eine Rastverbindung hergestellt, sondern durch die Magnete M1 und M2, wobei ein Magnet M1 an der Halterung 250 und der andere Magnet M2 an der relativ dazu verschwenkbaren Aufnahme 300 angeordnet ist.

In der ersten Position der Aufnahme gemäß Figur 4 a) ist die Magnetkraft ausreichend, um die Aufnahme 300 in der dargestellten Position zu halten.

Wird die Kraft F zu groß, übersteigt diese die Magnetkraft, woraufhin die Aufnahme 300 nach unten verschwenkt wird.

Unabhängig von dem genauen Mechanismus des Verschwenkens bzw. Abklappens der Aufnahme 300 durch eine Schwenkachse, Scharniere etc. wird sichergestellt, dass zu große auf die Aufnahme 300 wirkenden Kräfte bzw. Momente nicht oder nur abgemindert auf das Gehäuse 120 übertragen werden. Somit wird die Wahrscheinlichkeit für das Auftreten von Beschädigungen am Gerät und insbesondere an dem Gerätegehäuse verringert.

## Patentansprüche

1. Peritonealdialysegerät mit wenigstens einer Aufnahme (300) zur Fixierung eines oder mehrerer Abschnitte eines Schlauchsets zur Leitung von Dialyselösung von und/oder zu dem Patienten,
wobei wenigstens ein erste Schwenkachse (S1) vorgesehen ist, um die die Aufnahme (300) von einer ersten Position in eine zweite Position abklappbar ist,
wobei das Peritonealdialysegerät wenigstens ein Element (131) aufweist und die Aufnahme (300)
mittels einer Halterung (250) unmittelbar an dem Element (131) angeordnet ist, wobei sich die erste Schwenkachse (S1) an dem Element befindet und die Halterung (250) um die erste Schwenkachse (S1) abklappbar ist,
wobei das Peritonealdialysegerät wenigstens ein Gehäuse aufweist, an dem eine zweite Schwenkachse (S2) angeordnet ist, die nicht parallel zu der ersten Schwenkachse (S1) verläuft und um die das Element (131) mit der daran angeordneten Aufnahme (300) und die mit der daran angeordneten Aufnahme (300) in Verbindung stehende Halterung (250) relativ zum Gehäuse verdrehbar ist,
**dadurch gekennzeichnet, dass**
ein Haltemechanismus in Form einer Rastvorrichtung vorhanden ist und die Rastvorrichtung in Form eines federbelasteten Vorsprungs (260) und einer zugehörigen Aufnahme (262) für den federbelasteten Vorsprungs (260) derart ausgebildet ist, dass diese ein Abklappen der Halterung (250) von der ersten in die zweite Position nur bei Überschreiten eines Grenzwerts einer auf die Halterung bzw. auf die Aufnahme (300) wirkenden Kraft ermöglichen, wodurch der Vorsprung (260) entgegen der Federkraft eingedrückt wird und die Halterung (300) abgeklappt wird.

2. Peritonealdialysegerät nach Anspruch 1, **dadurch gekennzeichnet, dass** sich die erste Schwenkachse (S1) zwischen der Halterung (250) und dem Element (131) befindet.

3. Peritonealdialysegerät nach einem der vorhergehenden Ansprüche, **dadurch gekennzeichnet, dass** die erste (S1) Schwenkachse horizontal oder im Wesentlichen Horizontal angeordnet ist.

4. Peritonealdialysegerät nach einem der vorhergehenden Ansprüche, **dadurch gekennzeichnet, dass** die Aufnahme (300) in ihrer ersten Position horizontal oder im Wesentlichen Horizontal ausgerichtet ist.

5. Peritonealdialysegerät nach einem der vorhergehenden Ansprüche, **dadurch gekennzeichnet, dass** die zweite Schwenkachse (S2) vertikal oder im Wesentlichen vertikal verläuft.

6. Peritonealdialysegerät nach einem der vorhergehenden Ansprüche, **dadurch gekennzeichnet, dass** die erste und/oder die zweite Schwenkachse (S1, S2) unterhalb des Gehäuses (120) angeordnet ist.

## Claims

1. A peritoneal dialysis machine having at least one receptacle (300) for fixing one or more sections of a hose set for conducting dialysis solution from and/or to the patient,
wherein at least a first pivot axis (S1) is provided, about which the receptacle (300) can be pivoted from a first position to a second position,
wherein the peritoneal dialysis machine comprises at least one element (131) and the receptacle (300) is arranged directly at the element (131) by means of a holder (250), wherein the first pivot axis (S1) is located at the element and the holder (250) can be pivoted about the first pivot axis (S1),
wherein the peritoneal dialysis machine comprises at least one housing, on which a second pivot axis (S2) which does not extend in parallel to the first pivot axis (S1) is arranged and about which the element (131) with the receptacle (300) arranged thereon, and the holder (250) connected with the receptacle (300) arranged thereon can be rotated relative to the housing,
**characterized in that**
a holding mechanism in the type of latching device is present, and the latching device is configured in the type of a spring-loaded protrusion (260) and an associated receptacle (262) for the spring-loaded protrusion (260) in such a way that these allow a pivoting of the holder (250) from the first to the second position only when a force that acts on the holder, or the receptacle (300), exceeds a threshold value, whereby the protrusion (260) is pushed-in against the spring force and the receptacle (300) is pivoted.

2. Peritoneal dialysis machine according to claim 1, **characterized in that** the first pivot axis (S1) is located between the holder (250) and the element (131).

3. Peritoneal dialysis machine according to one of the preceding claims, **characterized in that** the first pivot axis (S1) is arranged horizontally or substantially horizontally.

4. Peritoneal dialysis machine according to one of the preceding claims, **characterized in that** the receptacle (300) in its first position is oriented horizontally or substantially horizontally.

5. Peritoneal dialysis machine according to one of the preceding claims, **characterized in that** the second pivot axis (S2) extends vertically or substantially vertically.

6. Peritoneal dialysis machine according to one of the preceding claims, **characterized in that** the first and/or second pivot axis (S1, S2) is arranged below the housing (120).

## Revendications

1. Appareil de dialyse péritonéale avec au moins un logement (300) servant à bloquer un ou plusieurs tronçons d'un ensemble de tuyaux flexibles servant à acheminer de la solution de dialyse depuis et/ou vers le patient,
dans lequel est prévu au moins un premier axe de pivotement (S1), autour duquel le logement (300) peut être rabattu depuis une première position dans une deuxième position,
dans lequel l'appareil de dialyse péritonéale présente au moins un élément (131) et le logement (300) est disposé directement au niveau de l'élément (131) au moyen d'une fixation (250), dans lequel le premier axe de pivotement (S1) se trouve au niveau de l'élément et la fixation (250) peut être rabattue autour du premier axe de pivotement (S1),
dans lequel l'appareil de dialyse péritonéale présente au moins un boîtier, au niveau duquel est disposé un deuxième axe de pivotement (S2), qui ne s'étend pas de manière parallèle par rapport eu premier axe de pivotement (S1) et autour duquel l'élément (131) avec le logement disposé (300) au niveau de celui-ci et la fixation (250) reliée au logement (300) disposé au niveau de celui-ci peuvent être tournés par rapport au boîtier,
**caractérisé en ce que**
un mécanisme de maintien est présent sous la forme d'un dispositif d'enclenchement, et le dispositif d'enclenchement est réalisé sous la forme d'une partie faisant saillie (260) soumise à une action de ressort et d'un logement (262) associé pour la partie faisant saillie (260) soumise à l'action d'un ressort de telle manière que ceux-ci permettent un rabattement de la fixation (250) de la première dans la deuxième position seulement en cas de dépassement d'une valeur limite d'une force agissant sur la fixation ou le logement (300), ce qui a pour effet que la partie faisant saillie (260) est enfoncée à l'encontre de la force de ressort et la fixation (300) est rabattue.

2. Appareil de dialyse péritonéale selon la revendication 1, **caractérisé en ce que** le premier axe de pivotement (S1) se trouve entre la fixation (250) et l'élément (131).

3. Appareil de dialyse péritonéale selon l'une quelconque des revendications précédentes, **caractérisé en ce que** le premier axe de pivotement (S1) est disposé de manière horizontale ou de manière sensiblement horizontale.

4. Appareil de dialyse péritonéale selon l'une quelconque des revendications précédentes, **caractérisé en ce que** le logement (300) est orienté dans sa première position de manière horizontale ou de manière sensiblement horizontale.

5. Appareil de dialyse péritonéale selon l'une quelconque des revendications précédentes, **caractérisé en ce que** le deuxième axe de pivotement (S2) s'étend de manière verticale ou de manière sensiblement verticale.

6. Appareil de dialyse péritonéale selon l'une quelconque des revendications précédentes, **caractérisé en ce que** le premier et/ou le deuxième axe de pivotement (S1, S2) sont disposés sous le boîtier (120).
